# EUROPEAN PATENT APPLICATION

(11) **EP 0 782 868 A1**
(43) Date of publication of application: **09.07.1997**
(21) Application number: 96100132.8
(22) Date of filing: 08.01.1996
(51) Int. Cl.: A61M 25/00

(54) **Packing system**

(71) Applicant: SCHNEIDER (EUROPE) AG, CH-8180 Bülach (CH)
(72) Inventor: Bodmer Andreas, CH.-8304 Wallisellen (CH)
(74) Representative: Misrachi, Alfred

(57) **Abstract**

The packaging system comprises a package housing 15 having a cup-like portion 16 including a substantially rigid grooved portion 20. An elongated medical appliance 1 having an active distal end portion 3 is engaged into a protective tube 10 formed into a coil 11 which is located in the cup-like portion 16. The active distal end portion 3 of the medical appliance 1 extends distally out of the protective tube 10 formed into a coil 11 and extends in the substantially rigid grooved portion 20. The protective tube 10 formed into a coil 11 is held in such a shape by a plurality of removable clamps 12. The system is low place consuming and allows easy flushing of the medical appliance.

## Description

This invention relates to a packaging system comprising an elongated medical appliance having a proximal end portion and an active distal end portion, said medical appliance being located in a protective tube arranged in a package housing.

Typically, elongated medical appliances, such as balloon catheters and stent delivery catheters as currently used in percutaneous transluminal angioplasty, are packaged by full insertion into a protective tube which is placed in a substantially soft pouch-like package housing. The package housing assures protection of the medical appliance against external pollution due to contaminating agents and the like, whereas the protective tube assures the mechanical protection of the medical appliance, and more particularly of the delicate balloon and/or stent structures forming its active distal end portion. Where the medical appliance is extremely long, such as is the case for example for balloon catheters of stent delivery catheters as used in percutaneous transluminal cardiovascular angioplasty, it has been found advisable to form the protective tube into a coil which is held in the coiled configuration by removable clamps, the medical appliance being also fully inserted into the protective tube formed into a coil which is also placed in a substantially soft pouch-like housing.

A problem with that kind of packaging systems is that it is difficult to flush the balloon and/or stent structures as normally required before intervention on a patient. As a matter of fact, the protective tube does not allow access to the balloon and/or stent structures. Accordingly, it is necessary either to cut a portion of the protective tube or to try to push the catheter a bit forward in order to give access to the balloon and/or stent configurations. To cut a portion of the protective tube is a time consuming and skill requiring operation because of the risk of damaging the delicate active distal end portion of the catheter. To push the catheter forward of the protective tube is not systematically possible depending on the relative lengths of the protective tube and catheter the proximal T-connector of which may act as an abutment preventing total or partial exit of the balloon and/or stent structures out of the protective tube. A further approach would be to completely withdraw the catheter out of the protective tube in order to have a good access to the balloon and/or stent configurations for flushing purposes. However, that would be an even more time consuming and skill demanding procedure because the length of the catheter makes it difficult to manipulate, which strongly raises the risk of damaging the balloon and/or stent structures and the risk of contamination of the catheter.

A further problem with such packaging systems is that their length makes them place consuming for storage and transit and cumbersome to manipulate for flushing purposes, with a serious risk of damage to and contamination of the medical appliance and more particularly its active distal end portion. Furthermore, if the medical appliance has some resiliency, the handling becomes even more difficult because of the spring effect which enhances the manipulation complication. Here again, the risk of damage or loss of sterilization of the active distal end portion would become hardly tolerable. When the protective tube is formed into a coil, the system becomes acceptable on the viewpoint of place consumption for storage and transit, but the problem of flushing remains unsolved, with the sole palliative measures as outlined hereabove.

Other packaging systems which do not make use of a protective tube also do not solve properly the problem of flushing the active distal end of the catheter. And they also remain hazardous to handle.

For example, the document US 3930580 shows a peelable pouch or tray composed of two superposed sheets interconnected by edge-strips of folded-over plastic material affixed to the exterior surfaces of the sheets. Access to the catheter located between the sheets is obtained by merely peeling one sheet back, thereby ripping the folded-over edge-strips at the juncture between the two sheets. If peeling starts conventionally at the proximal end of the catheter, the catheter must be fully extracted from the pouch to give access to its active distal end portion for flushing purposes, with the same drawbacks as outlined hereinbefore for the approach of fully withdrawing the catheter out of the tube. If peeling starts at the distal end of the catheter, flushing thereof would be possible, however, there is no way to properly hold and manipulate the catheter and handling thereof exposes the active distal end portion thereof to damage and loss of sterilization.

The purpose of this invention is to provide a packaging system avoiding the aforesaid problems. A further object of the invention is to provide a packaging system which is easy to handle without risk of damage or loss of sterilization for the medical appliance and more particularly its active distal end portion. Still a further object of the invention is to provide a packaging system that is versatile and does not require expensive manufacture and assembly proceedings.

To this effect, the invention complies with the definitions given in the claims.

Accordingly, where the active distal end portion of the medical appliance extends distally out of the protective tube in the package housing in a substantially rigid grooved portion thereof, the active distal end portion of the medical appliance may be flushed while the medical appliance is in the protective tube which assures an appropriate support for the medical appliance whereas the active distal end portion of the medical appliance remains well supported and fully accessible and protected in the grooved portion of the package housing. The protective tube and substantially rigid grooved portion of the package housing provide a combined mechanical protection of the medical appliance and of its active distal end portion and there is no need of unwanted manipulations of the active distal end portion of the medical appliance and no risk of hazardous handling which may cause damage or loss of sterilization. There is no need to cut any portion of the protective tube, nor is there any need to manipulate the medical appliance with respect to the protective tube to give access to the active distal end portion of the medical appliance. Inappropriate withdrawal of the medical appliance out of the protective tube is avoided. The medical appliance may be safely held and handled during flushing and the sole operation which is required before flushing is the mere opening of the package housing which may be achieved conventionally. When the medical appliance has some resiliency, there is no risk of loosing the handling control of the medical appliance due to any spring effect, whatever the length of the medical appliance. Handling can be safely achieved because of full control, access and protection of the medical appliance and of its active distal end portion.

The protective tube and the rigid grooved portion of the package housing may be devised simply and at low cost.

Where the system comprises shoulder means in the grooved portion for centering the active distal end portion of the medical appliance in said grooved portion, a further step is achieved in the positioning of the active distal end portion to assure the most even flushing all around the periphery of the active distal end portion.

The grooved portion may extend distally of the active distal end portion of the medical appliance in order to provide a handling room for removing a packaging safety wire inserted into the active distal end portion without unwanted manipulation of the active distal end portion of the medical appliance.

Where the protective tube and the grooved portion are located in a substantially rigid cup-like portion of the package housing, a solid frame is obtained for supporting the medical appliance in its protective tube as well the active distal end portion thereof, whereby flushing may be achieved without any need for touching the medical appliance.

In order to facilitate an efficient and rapid placement of the protective tube in the package housing, the packaging system may comprise boss means formed in said cup-like portion of the package housing for positioning the protective tube with respect to the grooved portion. Where such boss means define a groove structure in the cup-like portion of the package housing, whereas the protective tube is located in said groove structure, the advantage of an efficient and rapid placement of the tube is accompanied by the further advantage that the medical appliance gets an added mechanical protection against shocks and the like. And where the groove structure comprises a constricted area clamping a distal portion of the protective tube, the relative position of the protective tube with respect to the active distal end portion of the medical appliance may somewhat change in case of shocks and the like during transit or storage or handling without however influencing the correct and safe positioning of the active distal end portion in the rigid grooved portion of the package housing.

The protective tube may be formed into a coil to assure with the substantially rigid grooved portion a small spatial size together with fully protected accessibility to the active distal end portion of the medical appliance and the resulting packaging system becomes a low place consumption and readily functional item which requires a small room for storage and transit while taking advantage of the coil configuration of the protective tube which facilitates handling of the medical appliance during and after the flushing procedure. And to further enhance the safety arrangement with the protective tube formed into a coil, the system may comprise shoulder means for preventing rotational motion of the protective tube formed into a coil with respect to the grooved portion. Advantageously, such shoulder means are formed in the groove structure, whereby a very safe and fast positioning of the protective tube formed into a coil is achieved.

The packaging system may comprise means for holding the protective tube formed into a coil, whereby the protective tube may be advantageously made of currently available rectilinear tubing which is formed and held into a coil as appropriate. When the protective tube formed into a coil is separated from such holding means, the protective tube uncoils and the rectilinearity of the tubing allows easy placement and removal of the medical appliance in and from the tubing without any risk of unwanted bending of its active distal end portion. Within this frame, the means for holding the protective tube formed into a coil may be independent of the package housing to allow handling of the medical appliance in the protective tube formed into a coil as a unit independent from the package housing.

An economical way for manufacturing the means for holding the protective tube formed into a coil is achieved when such means comprise a plurality of clamps each of which is formed by a plurality of contiguous parallel channels respectively snapped on consecutive turns of the protective tube formed into a coil. With such a material the coil forming is quite easy, being sufficient to snap a portion of the protective tube in one of the channels of a clamp, and then to coil it and successively snap it in the corresponding channels of the other clamps, and so on up to termination of the coil as required.

These and other objects, features and advantages of the invention will become readily apparent from the following detailed description with reference to the accompanying drawings which show, diagrammatically and by way of example only, a preferred but still illustrative embodiment of the invention.

Figure 1 is a top plan view of a preferred embodiment of the packaging system in accordance with the present invention.

Figure 2 is a sectional view according to line II-II of Figure 1.

Figure 3 is a sectional view according to line III-III of Figure 1.

Figure 4 is a sectional view according to line IV-IV of Figure 1.

Figure 5 is a sectional view of an enlarged detail of Figure 1.

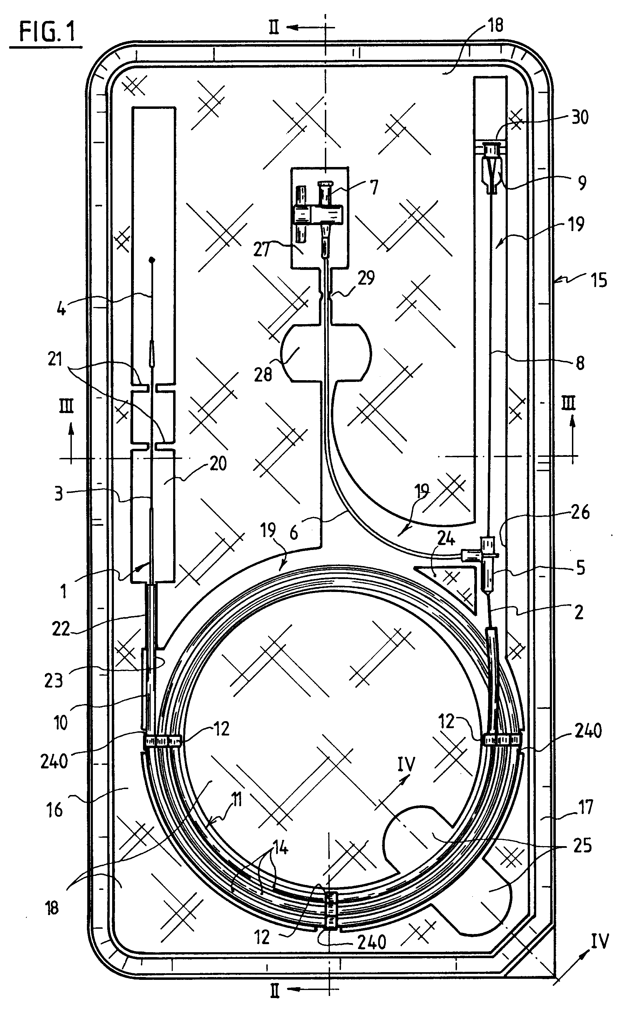

The packaging system shown in Figures 1 to 4 comprises an elongated medical appliance 1, in the example shown a stent delivery catheter, having a proximal end 2 and an active distal end portion 3, here a self-expandable stent. A packaging safety wire 4 is inserted into the active distal end portion 3. The proximal end portion 2 of the medical appliance 1 is conventionally connected to a T-connector 5 for manipulation and contrast fluid supply, such a T-connector 5 being joined via a pipe 6 to the usual stop-cock 7 for connection to a contrast fluid supply (not shown). The T-connector also allows passage to a control wire 8 for stent manipulation and the proximal end of which is attached to a luer-lock 9, as usual in the art.

The medical appliance 1 is inserted by its active distal end portion 3 into a protective tube 10 which is formed into a coil 11 which extends proximally substantially up to the T-connector 5 at the proximal end 2 of the medical appliance. The active distal end portion 3 of the medical appliance 1 extends distally out of the protective tube 10 formed into a coil 11.

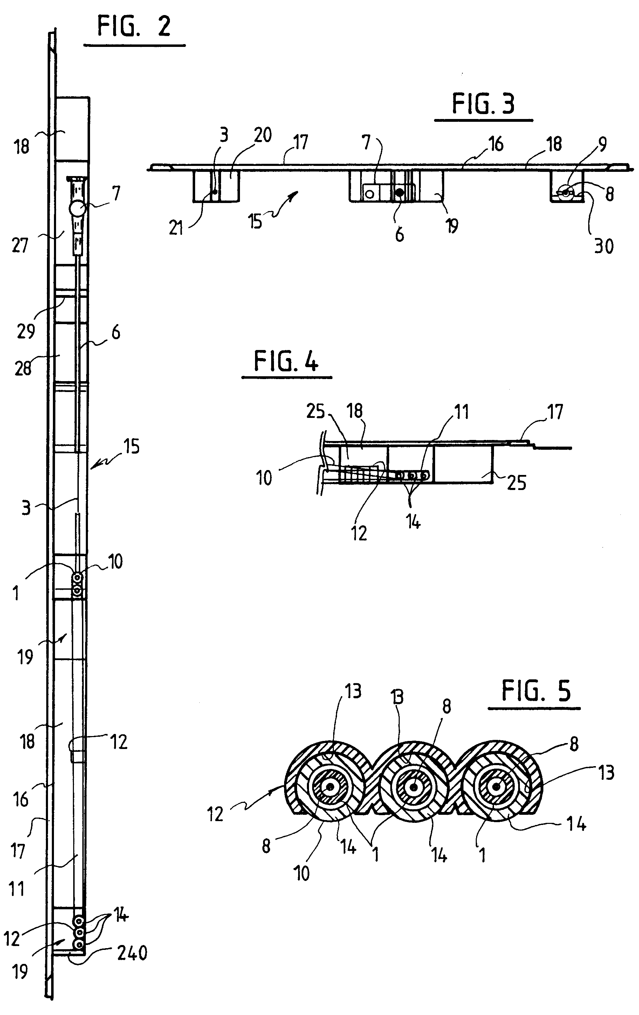

In the example shown, the protective tube 10 is made of currently available rectilinear tubing of flexible plastic material which is wound into a coil 11 and held in such a shape by means of a plurality of clamps 12. As shown in more detail in Figure 5, each of the clamps 12 is formed by three contiguous parallel channels 13 respectively snapped on consecutive turns 14 of the protective tube 10 formed into a coil 11.

The medical appliance 1 in its protective tube 10 formed into a coil 11 are arranged in a package housing 15 comprising a substantially rigid cup-like portion 16 surrounded by an edge 17 upon which is affixed a removable cover (not shown) which may be formed of a sheet of material allowing passage of a sterilizing means such as ethylene oxid or the like. The cup-like portion 16 may be made by blister technology, whereas the removable cover (not shown) may be preferably affixed to the edge 17. In the example shown, the cup-like portion 16 includes a plurality of bosses 18 defining a groove structure 19.

The groove structure 19 comprises a substantially rigid grooved portion 20 in which extends the active distal end portion 3 of the medical appliance 1. Shoulders 21 formed in the grooved portion 20 are centering the active distal end portion 3 in the grooved portion 20. The grooved portion 20 extends distally of the active distal end portion 3 of the medical appliance to allow easy grasping and withdrawal of the packaging safety wire 4 out of the distal active end portion 3 of the medical appliance 1.

The protective tube 10 formed into a coil 11 is positioned in the cup-like portion 16 with respect to the grooved portion 20 by one of the bosses 18 and the protective tube 10 formed into a coil 11 is thus located in the groove structure 19. The groove structure 19 comprises a constricted area 22 which slightly clamps the distal portion 23 of the protective tube 10.

The T-connector 5 is also positioned in the groove structure 19 and a shoulder 24 formed in the groove structure 19 prevents rotational motion of the protective tube 10 formed into a coil 11 with respect to the grooved portion 20, such a rotation prevention being achieved by abutment of the T-connector 5 against the shoulder 24 or against a wall 26 of the groove structure 19. Instead of or in addition to shoulder 24, prevention of rotational motion of the protective tube 10 formed into a coil 11 may be achieved by means of a plurality of shoulders 240 formed by the bosses 18 within the groove structure 19 (as shown in Figure 1) in order to provide an abutment for the clamps 12 holding the protective tube 10 formed into a coil 11. Such a configuration may be preferred to the shoulder 24 arrangement because of its direct action on the protective tube formed into a coil.

A pair of cavities 25 formed in the boss 18 positioning the coil 11 allow easy thumb and fore-finger grasp of the protective tube 10 formed into a coil 11 in the groove structure 19.

The pipe 6 connecting the T-connector 5 to the stop-cock 7 is also located in the groove structure 19 with the stop-cock 7 being positioned in a recess 27 formed therein by the bosses 18. A further recess 28, also formed in the groove structure 19 by the bosses 18, allows easy grasping of the pipe 6, whereas a constriction 29 of the bosses 18 slightly squeezes the pipe 6 for handling purposes.

The control wire 8 also extends in a portion of the groove structure 19, whereas the luer-lock 9, also located in said portion of the groove structure 19, may be immobilized therein between ribs 30 formed therein.

Variants are available without departing from the scope of the invention.

For example, instead of being held into a coil 11 by the clamps 12, the tube 10 formed into a coil 11 may be permanently preshaped into such a coil.

The clamps 12, may be independent from the package housing as shown, or they may be integrated into the package housing, the tube 10 being snapped therein for being formed into a coil.

And of course, the protective tube 10 may be straight instead of being formed into a coil 11.

## Claims

1. A packaging system comprising an elongated medical appliance (1) having a proximal end portion (2) and an active distal end portion (3), said medical appliance being located in a protective tube (10) arranged in a package housing (15), characterized in that the active distal end portion (3) of the medical appliance (1) extends distally out of the protective tube (10) in the package housing (15) in a substantially rigid grooved portion (20) thereof.

2. A packaging system according to claim 1, further comprising shoulder means (21) in said grooved portion (20) for centering the active distal end portion (3) of the medical appliance (1) in said grooved portion (20).

3. A packaging system according to claim 1 or 2, wherein said grooved portion (20) extends distally of the active distal end portion (3) of the medical appliance (1).

4. A packaging system according to any preceding claim, wherein said protective tube (10) and grooved portion (20) are located in a substantially rigid cup-like portion (16) of the package housing (15).

5. A packaging system according to claim 4, further comprising boss means (18) formed in said cup-like portion (16) of the package housing (15) for positioning said protective tube (10) with respect to said grooved portion (20).

6. A packaging system according to claim 5, wherein said boss means (18) define a groove structure (19) in the cup-like portion (16) of the package housing (15), and wherein said protective tube (10) is located in said groove structure (19).

7. A packaging system according to claim 6, wherein said groove structure (19) comprises a constricted area (22) clamping a distal portion (23) of the protective tube (10).

8. A packaging according to any preceding claim, wherein said protective tube (10) is formed into a coil (11).

9. A packaging system according to claim 8, further comprising shoulder means (24, 240) for preventing rotational motion of the protective tube (10) formed into a coil (11) with respect to said grooved portion (20).

10. A packaging system according to claims 6 and 9, wherein the shoulder means (240) are formed in the groove structure (19).

11. A packaging system according to any of claims 8 to 10, further comprising means (12) for holding the protective tube (10) formed into a coil (11).

12. A packaging system according to claim 11, wherein said means (12) for holding the protective tube (10) formed into a coil (11) are independent of the package housing (15).

13. A packaging system according to claim 11 or 12, wherein said means for holding the protective tube (10) formed into a coil (11) comprise a plurality of clamps (12) each of which is formed by a plurality of contiguous parallel channels (13) respectively snapped on consecutive turns (14) of the protective tube (10) formed into a coil (11).
